# DEMANDE DE BREVET EUROPEEN

(11) **EP 1 666 096 A1**
(43) Date de publication de la demande: **07.06.2006**
(21) Numéro de dépôt: 05292560.9
(22) Date de dépôt: 02.12.2005
(51) Int. Cl.: A61Q 5/06, A61Q 5/08, A61K 8/49, A61K 8/22

(54) **Composition pour la décoloration et la coloration simultanée des fibres kératiniques comprenant la quinoline ou un dérivé quinolinique**

(30) Priorité: 03.12.2004 FR 0452858
(71) Demandeur: L'OREAL, 75008 Paris (FR)
(72) Inventeur: Kravtchenko, Sylvain, 92600 Asnieres (FR); Bonnardel, Valérie, 92700 Colombes (FR)
(74) Mandataire: Prevel, Estelle Nicole

(57) **Abrégé**

La présente invention a pour objet une composition pour la décoloration et la coloration simultanée des fibres kératiniques comprenant au moins un colorant choisi parmi les dérivés quinoliniques et leurs sels d'addition, au moins un sel peroxygéné et au moins un agent alcalin, le procédé de décoloration et de coloration des fibres kératiniques mettant en oeuvre cette composition, ainsi que l'utilisation de cette composition pour la décoloration et la coloration simultanée des fibres kératiniques.

La composition conforme à la présente invention est particulièrement bien adaptée à des cheveux foncés. Elle présente une stabilité améliorée dans le temps et permet d'obtenir une coloration chromatique et tenace.

## Description

La présente invention a pour objet une composition pour la décoloration et la coloration simultanée des fibres kératiniques, et en particulier des fibres kératiniques humaines telles que les cheveux, comprenant au moins un colorant choisi parmi la quinoline, les dérivés quinoliniques et leurs sels d'addition, au moins un sel peroxygéné et au moins un agent alcalin.

Lorsqu'une personne souhaite changer radicalement de couleur de cheveux, notamment lorsqu'elle souhaite obtenir une couleur plus claire que sa couleur d'origine, il est souvent nécessaire de procéder à une décoloration, et éventuellement à une coloration des cheveux. Pour ce faire, il existe plusieurs méthodes.

La première méthode consiste à utiliser des produits éclaircissants à base d'ammoniaque et de peroxyde d'hydrogène. Ces produits peuvent éventuellement contenir des colorants ce qui permet d'éclaircir et de colorer simultanément les cheveux. Toutefois, les performances éclaircissantes de ces produits restent limitées, plus particulièrement pour des applications sur des cheveux à fonds foncés naturels et / ou colorés.

La deuxième méthode consiste à appliquer sur les cheveux une composition éclaircissante à base de sels peroxygénés tels que le persulfate et d'agents alcalins dans laquelle a été ajouté du peroxyde d'hydrogène au moment de l'emploi, afin d'obtenir un éclaircissement plus important. Ce type de produit est très satisfaisant et plus adapté à des fonds foncés, mais il ne conduit qu'à une gamme très restreinte de reflets. Il est alors nécessaire de corriger la nuance obtenue en appliquant dans un deuxième temps un produit de coloration sur les cheveux. Ce procédé en deux étapes présente l'inconvénient d'être relativement long.

Pour pallier cet inconvénient, il est connu d'ajouter à ces produits éclaircissants des colorants. Cette méthode permet de colorer et de décolorer simultanément la fibre capillaire. Le niveau d'éclaircissement étant important, elle est particulièrement bien adaptée à des fonds foncés naturels et / ou colorés. Cependant, il existe un nombre très réduit de colorants stables dans ces conditions très oxydantes ce qui limite la variété des reflets pouvant être obtenus. Par ailleurs, cette instabilité se traduit par une modification plus ou moins rapide du reflet durant l'application ce qui conduit à des résultats peu reproductibles.

De plus, la ténacité de ces colorants face aux agents extérieurs, en particulier la lumière et les shampooings, n'est pas satisfaisante.

Il a été proposé, dans le brevet US 5 688 291, la demande de brevet WO 02/074270 et le modèle d'utilité DE 203 03 559, des colorants directs de type anthraquinone, azo, triarylméthane, thiazine, quinone et nitro, qui sont stables dans ces milieux fortement oxydants. Ces colorants ne sont cependant pas satisfaisants en terme de chromaticité, de ténacité et de stabilité pendant le temps de pose.

Le but de la présente invention est de fournir de nouvelles compositions pour la décoloration et la coloration simultanée des fibres kératiniques, et en particulier des fibres kératiniques humaines telles que les cheveux, particulièrement bien adaptées à des fonds foncés, qui présentent une bonne stabilité dans le temps et permettent d'obtenir des colorations chromatiques et tenaces.

Ce but est atteint avec la présente invention qui a pour objet une composition pour la décoloration et la coloration simultanée des fibres kératiniques comprenant :
- au moins un colorant choisi parmi la quinoline, les dérivés quinoliniques et leurs sels d'addition ;
- au moins un sel peroxygéné ; et
- au moins un agent alcalin.

La composition conforme à la présente invention est particulièrement bien adaptée pour la décoloration et la coloration simultanée des cheveux foncés. Elle présente une stabilité améliorée dans le temps et permet d'obtenir une coloration chromatique. De plus, avec des concentrations adaptées en colorants selon l'invention, on peut obtenir des reflets pastels.

Cette coloration est résistante aux diverses agressions que peuvent subir les cheveux telles que les shampoings, les frottements, la lumière, les intempéries, la sueur et les déformations permanentes. Elle est aussi puissante, esthétique, et de plus peu sélective, c'est-à-dire qu'elle permet d'obtenir de faibles écarts entre différentes parties différemment sensibilisées d'un cheveu ou d'une chevelure.

La présente invention a également pour objet un procédé de décoloration et de coloration simultanée des fibres kératiniques mettant en oeuvre la composition conforme à l'invention, ainsi que des dispositifs à plusieurs compartiments pour la mise en oeuvre de ce procédé.

Un autre objet de la présente invention est l'utilisation de la composition conforme à l'invention pour la décoloration et la coloration simultanée des fibres kératiniques.

Selon un mode de réalisation particulier de l'invention, le ou les colorants sont choisis parmi les composés de formules (I) ou (I') suivantes et leurs sels d'addition : 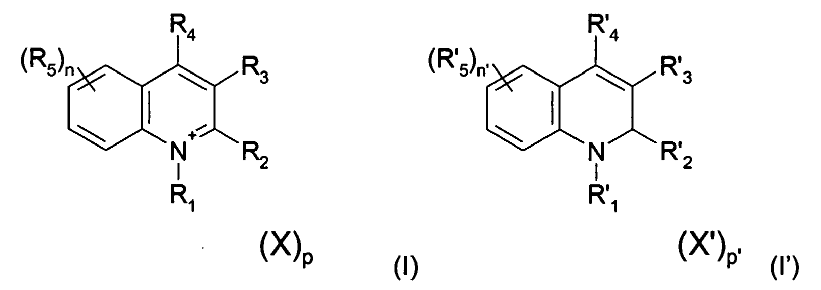 dans laquelle :
- R₁ et R₄ représentent, indépendamment l'un de l'autre :
   - un atome d'hydrogène ;
   - un radical alkyle ;
   - un radical hydroxyalkyle ;
- R₂ représente :
   - un atome d'hydrogène ;
   - un radical alkyle ;
- R₃ représente :
   - un atome d'hydrogène ;
   - un radical hétérocyclique à 5 ou 6 chaînons saturé ou insaturé éventuellement condensé avec un ou plusieurs cycles aromatiques ou non, hétérocycliques ou non, l'ensemble du groupement comprenant de 3 à 20 atomes de carbone et de 1 à 4 hétéroatomes, pouvant être substitué ou non substitué, et portant éventuellement une charge cationique ;
   - un radical alkyle ;
- R₅ représente :
   - un radical alkyle ;
   - un radical amino ;
   - un radical mono ou dialkylamino ;
   - un radical mono ou di(hydroxyalkyl)amino ;
   - un radical N,N-(alkyl)(hydroxyalkyl)amino ;
   - un radical sulfonato ;
- n est un nombre entier compris entre 0 et 4, étant entendu que lorsque n est supérieur ou égal à 2 alors les radicaux R₅ peuvent être identiques ou différents ;
- X désigne un atome ou un groupement d'atomes organique ou minéral chargé négativement permettant d'assurer la neutralité globale de la molécule ;
- p est un nombre entier égal à 0 ou 1 ;
- R'₁ R'₃, R'₄, R'₅, n', X' et p' ont respectivement la même définition que R₁, R₃, R₄, R₅, n, X et p ;
- R'₂ représente :
   - un atome d'hydrogène ;
   - un radical alkyle ;
   - un radical méthylidène disubstitué de formule (II) : 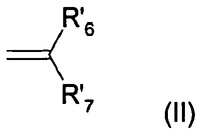
dans laquelle R'₆ et R'₇ représentent, indépendamment l'un de l'autre, un radical alkyle ; un radical hydroxyalkyle ; ou forment ensemble, avec l'atome de carbone auquel ils sont rattachés, un cycle à 5 ou 6 chaînons saturé ou insaturé dans lequel un ou plusieurs atomes de carbone peuvent être remplacés par un groupement carbonyle, éventuellement condensé avec un ou plusieurs cycles aromatiques ou non, hétérocycliques ou non, l'ensemble du groupement pouvant être ou non substitué par un ou plusieurs radicaux choisis parmi un radical alkyle, un radical alcoxy, un radical amino.

Dans le cadre de la présente invention, on entend par radical alkyle (alk) un radical linéaire ou ramifié comprenant de 1 à 6 atomes de carbone, par exemple un radical méthyle, éthyle, n-propyle, iso-propyle, n-butyle ou ter-butyle. Un radical alcoxy est un radical alk-O-, un radical mono ou dialkylamino est un radical (alk)ₙN- avec n = 1 ou 2, le radical alkyle étant tel que défini précédemment.

Un radical alkyle substitué est un radical alkyle mono ou polysubstitué. En particulier, un radical hydroxyalkyle est un radical alkyle qui peut être substitué par un ou plusieurs groupements hydroxy, un radical halogénoalkyle est un radical alkyle qui peut être substitué par un ou plusieurs groupements halogéno.

Un radical hétérocyclique à 5 ou 6 chaînons saturé ou insaturé éventuellement condensé avec un ou plusieurs cycles aromatiques ou non, hétérocycliques ou non, l'ensemble du groupement comprenant de 3 à 20 atomes de carbone et de 1 à 4 hétéroatomes et portant éventuellement une charge cationique peut être par exemple un radical quinolinium ou un radical indolium.

Un cycle à 5 ou 6 chaînons saturé ou insaturé dans lequel un ou plusieurs atomes de carbone peuvent être remplacés par un groupement carbonyle, éventuellement condensé avec un ou plusieurs cycles aromatiques ou non, hétérocycliques ou non, peut être par exemple un radical indanedione.

Dans toutes les significations précédentes et sauf indication contraire, lorsqu'un groupement est substitué, celui-ci est mono ou polysubstitué et les substituants sont choisis parmi les radicaux halogéno, hydroxy, alkyle, hydroxyalkyle, halogénoalkyle, alcoxy, amino, mono ou di(alkyl)amino, mono ou di(hydroxyalkyl)amino, carboxyle.

Selon un mode de réalisation particulier de l'invention, R₁ et R₄ représentent, indépendamment l'un de l'autre, un atome d'hydrogène ; un radical alkyle. A titre d'exemple, R₁ et R₄ sont choisis parmi un atome d'hydrogène ; un radical méthyle ; un radical éthyle.

Selon un mode de réalisation particulier de l'invention, R₂ représente un atome d'hydrogène ; un radical alkyle. A titre d'exemple, R₂ est choisi parmi un atome d'hydrogène ; un radical méthyle.

Selon un mode de réalisation particulier de l'invention, R₃ représente un atome d'hydrogène ; un radical quinolinium substitué ou non ; un radical 3H-indolium substitué ou non. A titre d'exemple, R₃ est choisi parmi un atome d'hydrogène ; un radical 1-méthyl-quinolinium ; un radical 1,3,3'-triméthyl-3H-indolium.

Selon un mode de réalisation particulier de l'invention, R₅ représente un radical mono ou di(alkyl)amino. A titre d'exemple, R₅ est un radical diméthylamino.

Selon un mode de réalisation particulier de l'invention, n est égal à 0 ou 1.

Selon un mode de réalisation particulier de l'invention, R'₁ et R'₄ représentent un atome d'hydrogène.

Selon un mode de réalisation particulier de l'invention, R'₂ représente un radical méthylidène disubstitué de formule (II). A titre d'exemple, R'₂ est un radical indanedione.

Selon un mode de réalisation particulier de l'invention, R'₃ représente un atome d'hydrogène.

Selon un mode de réalisation particulier de l'invention, R'₅ représente un radical sulfonato ; un radical alkyle. A titre d'exemple, R₅ est choisi parmi un radical sulfonato ; un radical méthyle.

Selon un mode de réalisation particulier de l'invention, n' est compris entre 1 et 4.

Selon un mode de réalisation préféré de l'invention, l'un au moins des radicaux R'₅ représente un radical sulfonato. De préférence, l'un au moins des radicaux R'₅ représente un radical sulfonato sous forme de sel de sodium.

A titre d'exemples de colorants utiles dans le cadre de la présente invention, on peut citer les composés suivants : 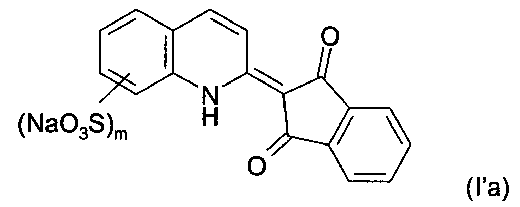 dans laquelle m est un nombre entier compris entre 1 et 3 ; 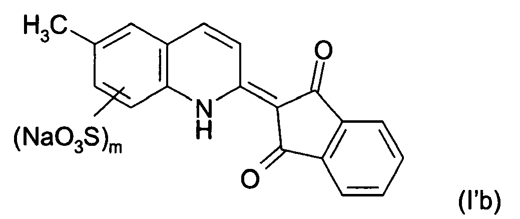 dans laquelle m est un nombre entier compris entre 1 et 3 ;

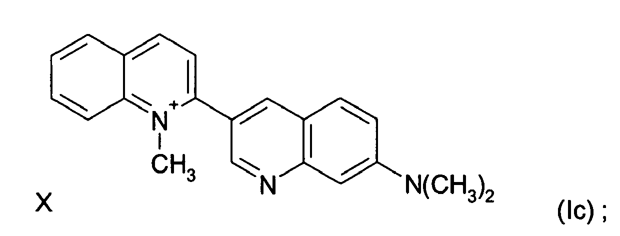

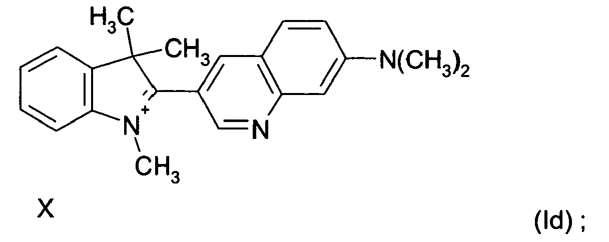

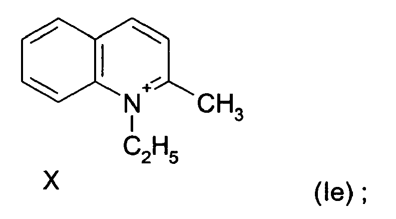

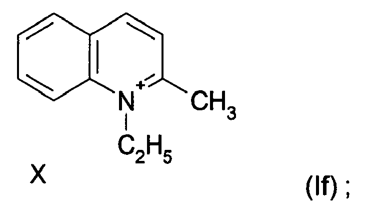

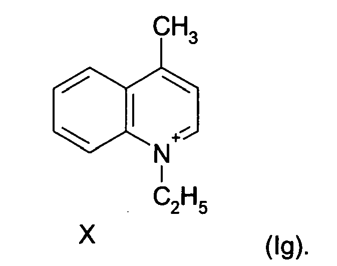

Selon un mode de réalisation particulier de l'invention, X désigne un alkylsulfonate ; un halogénure. A titre d'exemple, X est choisi parmi un méthylsulfonate ; un chlorure ; un iodure.

Selon un mode de réalisation préféré de l'invention, le ou les colorants utiles dans le cadre de l'invention sont choisis parmi les composés de formule (I') et leurs sels d'addition.

Selon un mode de réalisation préféré de l'invention, le ou les colorants sont choisis parmi les composés de formule (l'a) ; les composés de formule (l'b) ; et leurs sels d'addition.

De préférence, le colorant utile dans le cadre de la présente invention est le FOOD YELLOW 13.

D'une manière générale, les sels d'addition des dérivés quinoliniques utilisables dans le cadre de l'invention sont notamment choisis parmi les sels d'addition avec une base organique ou minérale, et en particulier les sels des métaux alcalins ou alcalino-terreux et les sels d'amines organiques telles que les alcanolamines. On préfère les sels de sodium.

La concentration en quinoline et / ou dérivés quinoliniques et / ou leurs sels d'addition dans la composition conforme à l'invention est généralement comprise entre 0,0001 et 10 % en poids, de préférence entre 0,001 et 8 %, et encore plus préféntiellement entre 0,01 et 5 % en poids du poids total de la composition.

Le ou les sels peroxygénés utiles à l'invention sont par exemple choisis parmi les persulfates, les perborates, les percarbonates, les peroxydes de métaux alcalins ou alcalino-terreux, et leurs mélanges. De préférence, on utilisera les persulfates et leurs mélanges, et plus préférentiellement les persulfates de sodium, de potassium et d'ammonium, et leurs mélanges.

La concentration en sels peroxygénés dans la composition conforme à l'invention est généralement comprise entre 10 et 70 % en poids, et de préférence entre 20 et 60 % en poids du poids total de la composition.

Le ou les agents alcalins utiles dans la composition de la présente invention sont par exemple choisis parmi l'urée, les sels d'ammonium comme le chlorure d'ammonium, le sulfate d'ammonium, le phosphate d'ammonium ou le nitrate d'ammonium, les silicates, les phosphates ou les carbonates de métaux alcalins ou alcalino-terreux, tels que le lithium, le sodium, le potassium, le magnésium, le calcium, le baryum, et leurs mélanges. De préférence, le ou les agents alcalins sont choisis parmi le chlorure d'ammonium, les silicates, les carbonates, et leurs mélanges.

La concentration en agents alcalins dans la composition conforme à l'invention est généralement comprise entre 0,01 et 40 % en poids, et de préférence entre 0,1 et 30 % en poids du poids total de la composition.

La composition conforme à l'invention peut se présenter sous forme de poudre ou de pâte. Avantageusement, la composition de l'invention se présente sous forme de pâte.

Dans le cas où la composition conforme à l'invention se présente sous forme de pâte, elle comprend de plus au moins une phase liquide inerte organique.

Par phase liquide, on entend au sens de la présente invention toute phase capable d'écoulement à température ambiante, généralement entre 15 °C et 40 °C, et à pression atmosphérique, sous l'action de son propre poids.

A titre d'exemple de phase liquide inerte, on peut citer les polydécènes de formule C₁₀ₙH_{[(20n)+2]} dans laquelle n varie de 3 à 9 et de préférence de 3 à 7, les esters d'alcools gras ou d'acides gras, les esters ou di-esters de sucres d'acides gras en C₁₂-C₂₄, les éthers cycliques ou les esters cycliques, les huiles de silicone, les huiles minérales ou les huiles végétales, ou leurs mélanges.

Les composés de formule C₁₀ₙ H_{[(20n)+2]} avec n variant de 3 à 9 répondent à l'appellation "polydécène" du Dictionnaire CTFA 7ème édition 1997 de la Cosmetic, Toiletry and Fragrance Association, USA, ainsi qu'à la même appellation I.N.C.I. aux USA et en Europe. Ce sont des produits d'hydrogénation des poly-1-décènes.

Parmi ces composés, on préfère selon l'invention ceux pour lesquels dans la formule, n varie de 3 à 7.

On peut citer à titre d'exemple le produit vendu sous la dénomination Silkflo® 366 NF Polydecene par la société Amoco Chemical, ceux vendus sous la dénomination Nexbase® 2002 FG, 2004 FG, 2006 FG et 2008 FG par la société Fortum.

En ce qui concerne les esters d'alcools gras ou d'acides gras, on peut citer à titre d'exemple :
- les esters de monoalcools inférieurs saturés linéaires ou ramifiés en C₃-C₆, avec des acides gras monofonctionnels en C₁₂-C₂₄, ces derniers pouvant être linéaires ou ramifiés, saturés ou insaturés et choisis notamment parmi les oléates, laurates, palmitates, myristates, béhénates, cocoates, stéarates, linoléates, linolénates, caprates, arachidonates, ou leurs mélanges comme notamment les oléo-palmitates, oléo-stéarates, palmito-stéarates. Parmi ces esters, on préfère plus particulièrement utiliser le palmitate d'isopropyle, le myristate d'isopropyle et le stéarate d'octyl dodécyle.
- les esters de monoalcools linéaires ou ramifiés en C₃-C₈, avec des acides gras bifonctionnels en C₈-C₂₄, ces derniers pouvant être linéaires ou ramifiés, saturés ou insaturés, comme par exemple le di-ester isopropylique de l'acide sébacique, appelé aussi sébaçate de di-isopropyle,
- les esters de monoalcools linéaires ou ramifiés en C₃-C₈, avec des acides gras bifonctionnels en C₂-C₈, ces derniers pouvant être linéaires ou ramifiés, saturés ou insaturés, comme par exemple l'adipate de di-octyle et le maléate de di-caprylyle,
- l'ester d'un acide trifonctionnnel comme le citrate de tri-éthyle.

En ce qui concerne les esters et di-esters de sucres d'acides gras en C₁₂-C₂₄, on entend par "sucre" des composés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres utilisables selon l'invention, on peut citer par exemple le sucrose (ou saccharose), le glucose, le galactose, le ribose, le fuctose, le maltose, le fructose, le mannose, l'arabinose, le xylose, le lactose, et leurs dérivés notamment alkylés, tels que les dérivés méthylés comme le méthylglucose.

Les esters de sucres et d'acides gras utilisables selon l'invention peuvent être choisis notamment dans le groupe comprenant les esters ou mélanges d'esters de sucres décrits ci-avant et d'acides gras en C₁₂-C₂₄, linéaires ou ramifiés, saturés ou insaturés.

Les esters peuvent être choisis parmi les mono-, di-, tri- et tétra-esters, les polyesters et leurs mélanges.

Ces esters peuvent être par exemple choisis parmi les oléates, laurates, palmitates, myristates, béhénates, cocoates, stéarates, linoléates, linolénates, caprates, arachidonates, ou leurs mélanges comme notamment les esters mixtes oléo-palmitates, oléo-stéarates, palmito-stéarates.

Plus particulièrement, on préfère utiliser les mono- et di- esters et notamment les mono- ou di- oléates, stéarates, béhénates, oléopalmitates, linoléates, linolénates, oléostéarates, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

En ce qui concerne les ethers cycliques et esters cycliques, conviennent notamment la γ-butyrolactone, le diméthyl isosorbide, ou le diisopropyl isosorbide.

Les huiles de silicone peuvent aussi être employées comme phase liquide organique inerte.

Plus particulièrement, les huiles de silicone convenables sont des fluides de silicones liquides et non volatiles de viscosité inférieure ou égale à 10 000 mPa.s à 25 °C, la viscosité des silicones étant mesurée selon la norme ASTM 445 Appendice C.

Les huiles de silicone sont définies plus en détail dans l'ouvrage de Walter NOLL "Chemistry and Technology of Silicones" (1968) - Academic Press.

Parmi les huiles de silicone utilisables selon l'invention, on peut citer notamment les huiles de silicones vendues sous les dénominations DC-200 fluid - 5 mPa.s, DC-200 fluid - 20 mPa.s, DC-200 fluid - 350 mPa.s, DC-200 fluid - 1000 mPa.s, DC-200 fluid - 10 000 mPa.s par la société Dow Corning.

Les huiles minérales peuvent aussi être utilisées comme phase liquide inerte organique, comme par exemple l'huile de paraffine.

Les huiles végétales peuvent aussi convenir, et notamment l'huile d'avocat, l'huile d'olive ou la cire liquide de jojoba.

De préférence, la phase liquide inerte organique est choisie dans le groupe formé par les polydécènes de formule C₁₀ₙH_{[(20n)+2]} dans laquelle n varie de 3 à 9 et de préférence de 3 à 7, les esters d'alcools gras ou d'acides gras, et leurs mélanges.

Selon un mode de réalisation particulier de l'invention, la teneur en phase liquide inerte organique varie de 5 à 60 % en poids, de préférence de 10 à 50 % en poids par rapport au poids de la pâte anhydre, et encore plus préférentiellement de 15 à 45 %.

Selon un mode de réalisation particulier de l'invention, la composition conforme à l'invention est anhydre.

Dans le cadre de la présente invention, une composition est anhydre lorsqu'elle présente une teneur en eau inférieure à 1 % en poids, et de préférence inférieure à 0,5 % en poids par rapport au poids total de la composition.

Selon un autre mode de réalisation particulier de l'invention, la composition conforme à l'invention comprend de plus du peroxyde d'hydrogène.

Le pH de la composition contenant du peroxyde d'hydrogène conforme à l'invention est généralement compris entre les valeurs 3 et 11. Il est de préférence compris entre 7 et 11.

La composition conforme à la présente invention peut également comprendre divers additifs classiquement utilisés en cosmétique.

La composition conforme à la présente invention peut ainsi comprendre des agents épaississants minéraux ou organiques, et en particulier des polymères épaississants associatifs ou non, anioniques, cationiques, non ioniques ou amphotères, des charges telles que des argiles, des liants tels que la vinylpyrrolidone, des lubrifiants comme les stéarates de polyol ou les stéarates de métaux alcalins ou alcalino-terreux, des silices hydrophiles ou hydrophobes, des pigments, des colorants autres que ceux de la présente invention, des agents matifiants comme les oxydes de titane ou encore des agents tensioactifs anioniques, non ioniques, cationiques, amphotères ou zwittérioniques, des agents antioxydants, des agents de pénétration, des agents séquestrants, des tampons, des agents dispersants, des agents filmogènes, des agents conservateurs, des agents opacifiants, des vitamines, des parfums, des polymères anioniques, cationiques, non ioniques, amphotères ou zwittérioniqes, des céramides, des agents de conditionnement tels que par exemple des silicones volatiles ou non volatiles, modifiées ou non modifiées.

Dans le cas où la composition conforme à l'invention comprend du peroxyde d'hydrogène, elle peut également comprendre des agents de contrôle du dégagement d'oxygène tels que le carbonate ou l'oxyde de magnésium.

Les additifs et les agents de contrôle du dégagement d'oxygène tels que définis précédemment peuvent être présents en quantité comprise pour chacun d'eux entre 0,01 et 40 % en poids, de préférence entre 0,1 et 30 % en poids par rapport au poids total de la composition.

Bien entendu, l'homme de l'art veillera à choisir ce ou ces éventuels composés complémentaires de manière telle que les propriétés avantageuses attachées intrinsèquement à la composition conforme à l'invention ne soient pas, ou substantiellement pas, altérées par la ou les adjonctions envisagées.

Le procédé de décoloration et de coloration simultanée conforme à la présente invention consiste à appliquer sur les fibres kératiniques une composition contenant du peroxyde d'hydrogène conforme à l'invention telle que définie précédemment.

La présente invention a également pour objet un dispositif à plusieurs compartiments, caractérisé par le fait qu'il contient au moins deux compositions dont le mélange conduit à une composition contenant du peroxyde d'hydrogène conforme à l'invention telle que définie précédemment.

Selon un mode de réalisation particulier de l'invention, le dispositif conforme à invention comporte un premier compartiment qui contient une composition (A) comprenant, dans un milieu approprié pour la teinture, au moins un colorant tel que défini précédemment, un deuxième compartiment qui contient une composition (B) anhydre comprenant au moins un sel peroxygéné et au moins un agent alcalin tels que définis précédemment, et un troisième compartiment qui contient une composition (E) aqueuse de peroxyde d'hydrogène.

Selon un autre mode de réalisation particulier de l'invention, le dispositif conforme à invention comporte un premier compartiment qui contient une composition (C) anhydre comprenant au moins un colorant tel que défini précédemment, au moins un sel peroxygéné et au moins un agent alcalin tels que définis précédemment, et un deuxième compartiment qui contient une composition (E) aqueuse de peroxyde d'hydrogène.

Selon un autre mode de réalisation particulier de l'invention, le dispositif conforme à invention comporte un premier compartiment qui contient une composition (B) anhydre comprenant au moins un sel peroxygéné et au moins un agent alcalin tels que définis précédemment, et un deuxième compartiment qui contient une composition (D) comprenant, dans un milieu approprié pour la teinture, au moins un colorant tel que défini précédemment et du peroxyde d'hydrogène.

Le milieu approprié pour la teinture des compositions (A) et (D) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids par rapport au poids total de la composition tinctoriale, et plus préférentiellement encore entre 5 et 30 % en poids environ.

La composition (A), encore appelée "booster", peut être formulée à pH acide, neutre ou alcalin, le pH pouvant varier entre 3 et 12 environ et de préférence entre 4 et 11 environ.

La composition (D) présente de préférence un pH inférieur à 7, le pH acide garantissant la stabilité du peroxyde d'hydrogène dans cette composition.

Les compositions (A) et (D) peuvent se présenter sous des formes diverses, telles que sous forme de liquides, de crèmes, de gels, ou sous toute autre forme appropriée pour réaliser une teinture des fibres kératiniques.

Les compositions (B) et (C) anhydres peuvent se présenter sous forme de poudre ou de pâte. Dans ce cas-là, elles comprennent de plus une phase liquide inerte organique telle que définie précédemment.

La composition (E) aqueuse de peroxyde d'hydrogène présente de préférence un pH inférieur à 7, le pH acide garantissant la stabilité du peroxyde d'hydrogène dans cette composition.

Les compositions (A), (B), (C), (D) et (E) peuvent également renfermer divers additifs classiquement utilisés en cosmétique tels que ceux qui sont décrits précédemment.

Les compositions (E) et (D) peuvent de plus comprendre des agents de contrôle du dégagement d'oxygène tels que définis précédemment.

Le dispositif conforme à la présente invention peut être équipé d'un moyen permettant de délivrer sur les cheveux le mélange souhaité, tel que les dispositifs décrits dans le brevet FR-2 586 913 au nom de la demanderesse.

A partir de ce dispositif, il est possible de décolorer et de colorer simultanément les fibres kératiniques à partir d'un procédé conforme à l'invention tel que défini précédemment.

La présente invention a aussi pour objet l'utilisation pour la décoloration et la coloration simultanée des fibres kératiniques d'une composition conforme à l'invention telle que définie précédemment.

Les exemples qui suivent servent à illustrer l'invention sans toutefois présenter un caractère limitatif.

### EXEMPLE

Le Food Yellow 13 est solubilisé dans une solution hydroalcoolique (80 / 20) à la concentration de 3,5 g %. On obtient ainsi le booster A.
Cette solution est ajoutée juste avant utilisation à un mélange de la poudre Platine Précision B comprenant 51,5 % d'un mélange de persulfates de sodium, de potassium et de magnésium en présence de 4,2 % d'un mélange de métasilicate de sodium et de chlorure d'ammonium avec un oxydant E constitué par une composition aqueuse de peroxyde d'hydrogène à 40 volumes. Les proportions du mélange poudre décolorante B / oxydant E / booster A sont respectivement 1/2/0,5.
Une partie de ce mélange est appliqué immédiatement sur une mèche de cheveux naturels 90 % blancs de 1 g ainsi que sur une mèche de cheveux naturels chatains de 2,7 g.
Le restant de ce mélange est appliqué 20 minutes après sur une mèche de cheveux naturels 90 % blancs de 1 g ainsi que sur une mèche de cheveux naturels chatains de 2,7 g.
Dans tous les cas, les conditions sont identiques. Le rapport de bain est égal à 10.
Après un temps de pose de 30 minutes, les mèches sont rincées puis shampooinées, rincées à nouveau et séchées.
Les résultats sont décrits dans le tableau ci-dessous.

| **REFLETS OBTENUS SUITE A L'APPLICATION DES COMPOSITIONS DE l'INVENTION** | | |
|---|---|---|
| | Cheveux naturels 90 % blancs | Cheveux naturels chatains |
| **Application immédiate du mélange** | Jaune pâle | Doré cuivré |
| **Application différée du mélange** | Jaune pâle | Doré cuivré |

Les cheveux naturels 90 % blancs sont utilisés ici pour exacerber l'éventuelle modification de reflet.
On constate que l'on obtient le même reflet dans le cas où l'application du mélange est immédiate et dans le cas où elle est différée.
Ces résultats montrent que les compositions conformes à l'invention sont stables dans le temps.

## Revendications

1. Composition pour la décoloration et la coloration simultanée des fibres kératiniques comprenant :
- au moins un colorant choisi parmi la quinoline, les dérivés quinoliniques et leurs sels d'addition ;
- au moins un sel peroxygéné ; et
- au moins un agent alcalin.

2. Composition selon la revendication 1, dans laquelle le ou les colorants sont choisis parmi les composés de formules (I) ou (I') suivantes et leurs sels d'addition : 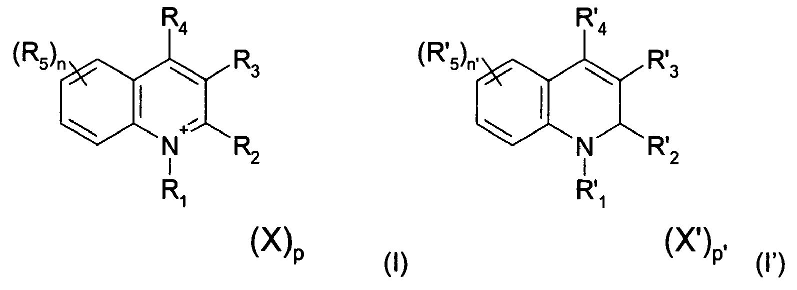 dans laquelle :
• R₁ et R₄ représentent, indépendamment l'un de l'autre :
- un atome d'hydrogène ;
- un radical alkyle ;
- un radical hydroxyalkyle ;
• R₂ représente :
- un atome d'hydrogène ;
- un radical alkyle ;
• R₃ représente :
- un atome d'hydrogène ;
- un radical hétérocyclique à 5 ou 6 chaînons saturé ou insaturé éventuellement condensé avec un ou plusieurs cycles aromatiques ou non, hétérocycliques ou non, l'ensemble du groupement comprenant de 3 à 20 atomes de carbone et de 1 à 4 hétéroatomes, pouvant être substitué ou non substitué, et portant éventuellement une charge cationique ;
- un radical alkyle ;
• R₅ représente :
- un radical alkyle ;
- un radical amino ;
- un radical mono ou dialkylamino ;
- un radical mono ou di(hydroxyalkyl)amino ;
- un radical N,N-(alkyl)(hydroxyalkyl)amino ;
- un radical sulfonato ;
• n est un nombre entier compris entre 0 et 4, étant entendu que lorsque n est supérieur ou égal à 2 alors les radicaux R₅ peuvent être identiques ou différents ;
• X désigne un atome ou un groupement d'atomes organique ou minéral chargé négativement permettant d'assurer la neutralité globale de la molécule ;
• p est un nombre entier égal à 0 ou 1 ;
• R'₁ R'₃, R'₄, R'₅, n', X' et p' ont respectivement la même définition que R₁, R₃, R₄, R₅, n, X et p ;
• R'₂ représente :
- un atome d'hydrogène ;
- un radical alkyle ;
- un radical méthylidène disubstitué de formule (II) : 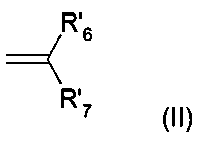
dans laquelle R'₆ et R'₇ représentent, indépendamment l'un de l'autre, un radical alkyle ; un radical hydroxyalkyle ; ou forment ensemble, avec l'atome de carbone auquel ils sont rattachés, un cycle à 5 ou 6 chaînons saturé ou insaturé dans lequel un ou plusieurs atomes de carbone peuvent être remplacés par un groupement carbonyle, éventuellement condensé avec un ou plusieurs cycles aromatiques ou non, hétérocycliques ou non, l'ensemble du groupement pouvant être ou non substitué par un ou plusieurs radicaux choisis parmi un radical alkyle, un radical alcoxy, un radical amino.

3. Composition selon la revendication 2, dans laquelle R₁ et R₄ représentent, indépendamment l'un de l'autre, un atome d'hydrogène ; un radical alkyle.

4. Composition selon la revendication 3, dans laquelle R₁ et R₄ représentent, indépendamment l'un de l'autre, un atome d'hydrogène ; un radical méthyle ; un radical éthyle.

5. Composition selon l'une quelconque des revendications 2 à 4, dans laquelle R₂ représente un atome d'hydrogène ; un radical alkyle.

6. Composition selon la revendication 5, dans laquelle R₂ représente un atome d'hydrogène ; un radical méthyle.

7. Composition selon l'une quelconque des revendications 2 à 6, dans laquelle R₃ représente un atome d'hydrogène ; un radical quinolinium substitué ou non ; un radical 3H-indolium substitué ou non.

8. Composition selon la revendication 7, dans laquelle R₃ représente un atome d'hydrogène ; un radical 1-méthyl-quinolinium ; un radical 1,3,3'-triméthyl-3H-indolium.

9. Composition selon l'une quelconque des revendications 2 à 8, dans laquelle R₅ représente un radical mono ou di(alkyl)amino.

10. Composition selon la revendication 9, dans laquelle R₅ représente un radical diméthylamino.

11. Composition selon l'une quelconque des revendications 2 à 10, dans laquelle n est égal à 0 ou 1.

12. Composition selon la revendication 2, dans laquelle R'₁ et R'₄ représentent un atome d'hydrogène.

13. Composition selon la revendication 2 ou 12, dans laquelle R'₂ représente un radical méthylidène disubstitué de formule (II).

14. Composition selon la revendication 13, dans laquelle R'₂ représente un radical indanedione.

15. Composition selon l'une quelconque des revendications 2 et 12 à 14, dans laquelle R'₃ représente un atome d'hydrogène.

16. Composition selon l'une quelconque des revendications 2 et 12 à 15, dans laquelle R'₅ représente un radical sulfonato ; un radical alkyle.

17. Composition selon la revendication 16, dans laquelle R'₅ représente un radical sulfonato ; un radical méthyle.

18. Composition selon l'une quelconque des revendications 2 et 12 à 17, dans laquelle n' est compris entre 1 et 4.

19. Composition selon la revendication 18, dans laquelle l'un au moins des radicaux R'₅ représente un radical sulfonato.

20. Composition selon la revendication 19, dans laquelle l'un au moins des radicaux R'₅ représente un radical sulfonato sous forme de sel de sodium.

21. Composition selon l'une quelconque des revendications 1 à 20, dans laquelle le ou les colorants sont choisis parmi les composés suivants : 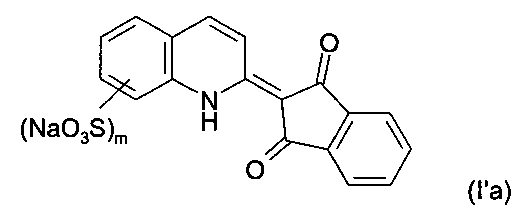 dans laquelle m est un nombre entier compris entre 1 et 3 ; 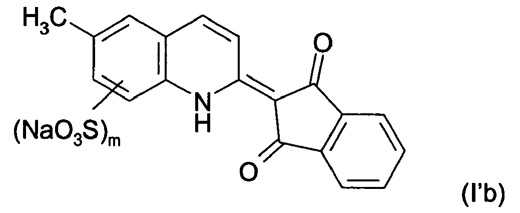 dans laquelle m est un nombre entier compris entre 1 et 3 ;

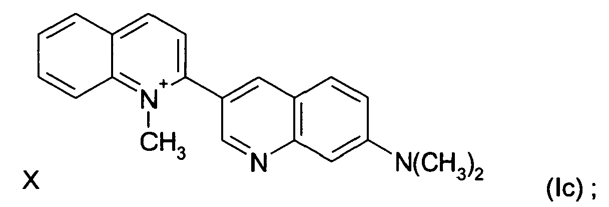

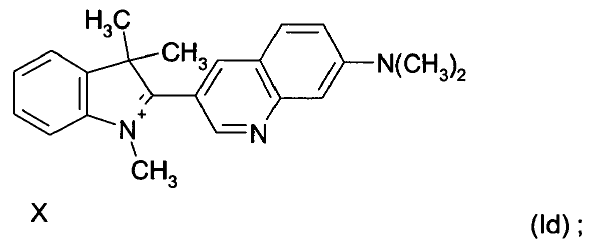

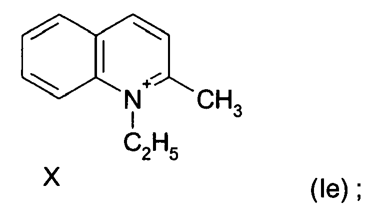

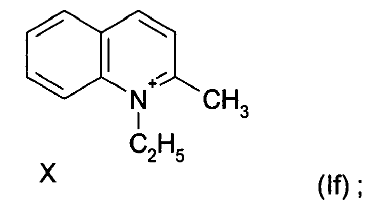

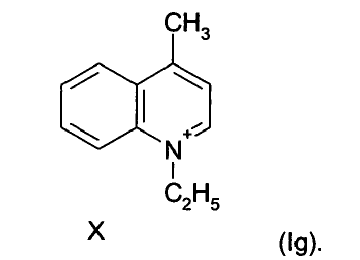

22. Composition selon l'une quelconque des revendications 2 à 21, dans laquelle X désigne un alkylsulfonate ; un halogénure.

23. Composition selon la revendication 22, dans laquelle X désigne un méthylsulfonate ; un chlorure ; un iodure.

24. Composition selon l'une quelconque des revendications 2 à 23, dans laquelle le ou les colorants sont choisis parmi les composés de formule (I') et leurs sels d'addition.

25. Composition selon l'une quelconque des revendications 21 à 24, dans laquelle le ou les colorants sont choisis parmi les composés de formule (l'a) ; les composés de formule (l'b) ; et leurs sels d'addition.

26. Composition selon la revendication 25, dans laquelle le colorant est le FOOD YELLOW 13.

27. Composition selon l'une quelconque des revendications précédentes, dans laquelle la concentration en quinoline et / ou dérivés quinoliniques et / ou leurs sels d'addition est comprise entre 0,0001 et 10 % en poids du poids total de la composition.

28. Composition selon l'une quelconque des revendications précédentes, dans laquelle le ou les sels peroxygénés sont choisis parmi les persulfates, les perborates, les percarbonates, les peroxydes de métaux alcalins ou alcalino-terreux, et leurs mélanges.

29. Composition selon la revendication 28, dans laquelle le ou les sels peroxygénés sont choisis parmi les persulfates et leurs mélanges.

30. Composition selon la revendication 29, dans laquelle le ou les sels peroxygénés sont choisis parmi le persulfate de sodium, le persulfate de potassium, le persulfate d'ammonium, et leurs mélanges.

31. Composition selon l'une quelconque des revendications précédentes, dans laquelle la concentration en sels peroxygénés est comprise entre 10 et 70 % en poids du poids total de la composition.

32. Composition selon l'une quelconque des revendications précédentes, dans laquelle le ou les agents alcalins sont choisis parmi l'urée, le chlorure d'ammonium, le sulfate d'ammonium, le phosphate d'ammonium, le nitrate d'ammonium, les silicates, les phosphates ou les carbonates de métaux alcalins ou alcalino-terreux, et leurs mélanges.

33. Composition selon l'une quelconque des revendications précédentes, dans laquelle la concentration en agents alcalins est comprise entre 0,01 et 40 % en poids du poids total de la composition.

34. Composition selon l'une quelconque des revendications précédentes, comprenant de plus au moins une phase liquide inerte organique.

35. Composition selon la revendication 34, dans laquelle la ou les phases liquides inertes organiques sont choisies parmi par les polydécènes de formule C₁₀ₙH_{[(20n)+2]} dans laquelle n varie de 3 à 9, les esters d'alcools gras ou d'acides gras, les esters ou di-esters de sucres d'acides gras en C₁₂-C₂₄, les éthers cycliques ou les esters cycliques, les huiles de silicone, les huiles minérales, les huiles végétales, et leurs mélanges.

36. Composition selon la revendication 35, dans laquelle la ou les phases liquides inertes organiques sont choisies parmi les polydécènes de formule C₁₀ₙH_{[(20n)+2]} dans laquelle n varie de 3 à 9, les esters d'alcools gras ou d'acides gras, et leurs mélanges.

37. Composition selon l'une quelconque des revendications 34 à 36, dans laquelle la concentration en phases liquides inertes organiques est comprise entre 5 et 60 % en poids du poids total de la composition.

38. Composition selon l'une quelconque des revendications précédentes, **caractérisée en ce qu'**elle est anhydre.

39. Composition selon l'une quelconque des revendications 1 à 37, comprenant de plus du peroxyde d'hydrogène.

40. Procédé de décoloration et de coloration simultanée des fibres kératiniques, **caractérisé par le fait que** l'on applique sur lesdites fibres kératiniques une composition telle que définie à la revendication 39.

41. Dispositif à plusieurs compartiments, comprenant au moins deux compositions dont le mélange conduit à une composition telle que définie à la revendication 39.

42. Dispositif selon la revendication 41, dans lequel un premier compartiment contient une composition (A) comprenant, dans un milieu approprié pour la teinture, au moins un colorant tel que défini à l'une quelconque des revendications 1 à 26, un deuxième compartiment contient une composition (B) anhydre comprenant au moins un sel peroxygéné tel que défini à l'une quelconque des revendications 1 et 28 à 30 et au moins un agent alcalin tel que défini à la revendication 1 ou 32, et un troisième compartiment contient une composition (E) aqueuse de peroxyde d'hydrogène.

43. Dispositif selon la revendication 41, dans lequel un premier compartiment contient une composition (C) anhydre comprenant au moins un colorant tel que défini à l'une quelconque des revendications 1 à 26, au moins un sel peroxygéné tel que défini à l'une quelconque des revendications 1 et 28 à 30 et au moins un agent alcalin tel que défini à la revendication 1 ou 32, et un deuxième compartiment contient une composition aqueuse (E) de peroxyde d'hydrogène.

44. Dispositif selon la revendication 41, dans lequel un premier compartiment contient une composition (B) anhydre comprenant au moins un sel peroxygéné tel que défini à l'une quelconque des revendications 1 et 28 à 30 et au moins un agent alcalin tel que défini à la revendication 1 ou 32, et un deuxième compartiment contient une composition (D) comprenant, dans un milieu approprié pour la teinture, au moins un colorant tel que défini à l'une quelconque des revendications 1 à 26 et du peroxyde d'hydrogène.

45. Utilisation pour la décoloration et la coloration simultanée d'une composition telle que définie à l'une quelconque des revendications 1 à 39.
